# EUROPEAN PATENT APPLICATION

(11) **EP 1 099 698 A1**
(43) Date of publication of application: **16.05.2001**
(21) Application number: 00203966.7
(22) Date of filing: 13.11.2000
(51) Int. Cl.: C07D 321/00, C11B 9/00

(54) **Macrocyclic oxalactones ( dioxacycloalkan-ones ) and their use as perfumes**

(30) Priority: 12.11.1999 EP 99203787
(71) Applicant: PFW AROMA CHEMICALS B.V., 3771 ME Barneveld (NL)
(72) Inventor: van Manen, Anton Pieter Johan, 3881 CM Putten (NL); Phillipse, Hendrikus Johannes Franciskus, 6666 HT Heteren (NL); Lenselink, Willem, 3781 VC Voorthuizen (NL)
(74) Representative: Prins, Adrianus Willem

(57) **Abstract**

The invention relates to a novel class of macrocyclic oxalactones which are highly useful for use as perfumes. The compounds according to the invention have a very desirable fresh and floral musk odor in combination with other advantageous properties, such as stability, and substantivity.

## Description

The invention relates to new chemical compounds useful as perfumes or as components of perfumes. More specifically it relates to macrocyclic lactones comprising an additional ether moiety in the ring.

There is a continuing search for materials having useful and desirable perfumery fragrance characteristics. These materials are sought either as replacements for naturally occurring or synthetic compounds or as totally new scents or odor notes in their own rights. For practicability reasons such materials should possess other favorable properties e.g. stability in applications and human and environmentally safety, all in addition to their useful odor notes.

Fragrance chemicals with musk odor properties are widely used in perfumery, not only in fine fragrances but particularly in functional perfumery, such as cosmetics, soap, detergent, shampoo perfumery, fabric care products, household cleaners, air refresheners and the like. They are not only preferred because of their irreplaceable odor tonality. In addition, many of them exhibit other favorable properties like odor and chemical stability in the application and under the conditions of use, which make them specifically useful in harsh environments, and the property of substantivity which make them well noticeable after treatment of an object with the perfumed functional product.

It should be understood that the commercial applicability of fragrance chemicals in applications, apart from these special properties, is determined by the economic effectivity, i.e. the contribution to the cost of the consumer product at an effective dosage level. For that reason, of the three main classes of musk fragrance chemicals known, the macrocyclic musks, the nitromusks and the polycyclic musks, the latter two classes are particularly effective in the perfumery of the lower end market segments of functional products. However, certain toxicological properties and low biodegradability properties in nitromusk class representatives and also low biodegradability properties in representatives of the polycyclic musk class, initiated a search for alternatives, especially for the polycyclic musks which are nowadays widely applied in the lower end market segments of functional products. Macrocyclic musks are generally not considered as suitable alternatives in this respect, because of unfavorable economic effectivity and/or unfavorable stability and/or substantivity properties and also not because of their additional, animalic odor tonalities.

In the European patent application 0 884 315 it has been proposed to employ 14 to 17 membered, 4-methyl substituted 1,7-dioxacycloalkan-8-ones or 14 to 16 membered, 4,4-dimethyl substituted 1,7-dioxacycloalkan-8-ones as fragrances. It is mentioned that these compounds are of acceptable toxicity and show improved biodegradability. They do, however, not have an odor of the desired quality.

It is an object of the present invention to provide a series of practicable synthetic materials, which possess very useful odors of the musky, woody and floral type and which are highly effective in the perfumery of a wide scope of products, including functional products and luxury products. It is further desired that the series of compounds is biodegradable and has an acceptable toxicity. Furthermore, the compounds should be very stable, enabling their application in compositions for use under harsh conditions.

Surprisingly, it has now been found that certain macrocyclic lactones comprising an additional ether moiety in the ring meet the above requirements. The novel macrocyclic oxalactones according to the invention are represented by the generic formula (I) 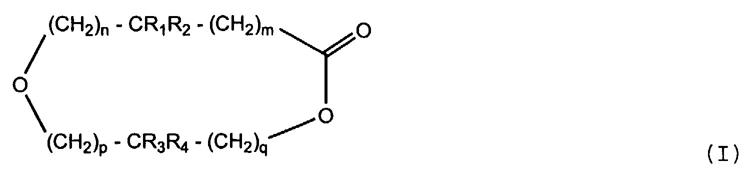 wherein
n and m represent an integer with the value 0, 1, 2, 3 or 4 the sum of n and m being 4 or less;
p and q represent an integer with the value 0 through 15 the sum of p and q being 15 or less;
the sum of n, m, p and q is 9, 10, 11, 12, 13, 14 or 15; and
R₁, R₂, R₃ and R₄ independently represent hydrogen or a lower alkyl group,
with the proviso that
when R₁, R₂, R₃ and R₄ all represent hydrogen:
p + q ≤ 11 if n + m = 0,
p + q = 11 if n + m = 1,
n + m + p + q ≠ 12 if n + m = 2,
n + m + p + q is not 11 or 12 if n + m = 3,
when R₁ represents a methyl group and R₂, R₃ and R₄ all represent hydrogen,
n + m + p + q ≠ 9 if n + m = 0.

Preferably, the lower alkyl group is chosen such that R₁, R₂, R₃ and R₄ combined do not have more than eight carbon atoms. It is further preferred that one of R₁ and R₂ is a hydrogen atom. Likewise, it is preferred that one of R₃ and R₄ is a hydrogen atom. In a highly preferred embodiment, the lower alkyl group has one or two carbon atoms, and preferably is a methyl group. In another highly preferred embodiment, all of R₁, R₂, R₃ and R₄ are hydrogen.

Compounds which are particularly preferred according to the invention are those wherein the sum of n and m is zero and the sum of p and q is 9 or 10. These compounds have been found to have a very well-balanced property profile in combination with a highly desirable odor. Other particularly preferred compounds are those wherein the sum of p and q is 11 and wherein the sum of n, m, p and q is 11, 12 or 13. Specifically preferred is a compound wherein n and m are zero, and the sum of p and q is 11. These compounds not only combine a very well-balanced property profile with a highly desirable odor, but can further be prepared in a very convenient and cost effective manner.

It will be apparent that the present macrocyclic oxalactones can exist in a variety of positional, stereoisomeric and enantiomeric forms and it is intended that these be included within the structural formulae.

The present macrocyclic oxalactones exhibit very useful odor nuances with unexpected clean, fresh, and floral musk character, with a clear polycyclic musk odor signature. Unexpectedly, they outperform their analogues known in the art in terms of polycyclic musk odor resemblance, radiance and odor stability and substantivity. This makes them specially suitable to be applied in fabric care, functional care and personal care consumer products. They can be used as fragrances per se or as components of a fragrance composition.

The term fragrance composition is used to denote a mixture of compounds including, for example, natural oils, synthetic oils, alcohols, aldehydes, ketones, esters, lactone, ethers, hydrocarbons, nitriles and other classes of chemical compounds which are admixed so that the combined odors of the individual components produce a pleasant or desired fragrance. Such fragrance compositions or the novel compounds of the invention alone can be used in conjunction with carriers, vehicles or solvents containing also as needed, dispersants, emulsifiers, surface-active agents, aerosols propellants odor release influencing agents and the like. In fragrance compositions the individual components contribute their particular olfactory characteristics, but the overall effect of the composition is the sum of the effect of each ingredient. Thus, the compounds of the invention can be used to alter, enhance, or reinforce the aroma characteristics of other natural or synthetic materials making up the fragrance composition, for example, by highlighting or moderating the olfactory reaction contributed by another ingredient or combination of ingredients.

The amount of the compounds of the invention which will be effective for a certain application depends on many factors including the characteristics of the other ingredients, their amounts and the effects which are desired. As little as 0.1% by weight of compounds of this invention can be used to alter the effect of a fragrance composition. The amount employed will depend on considerations of cost, nature of end product, the effect desired in the finished product, and the particular fragrance sought, but will usually not be more than about 50% by weight. The compounds disclosed herein can be used in a wide variety of applications, by way of example but not limited thereto, detergents and soaps, air fresheners, perfumes, colognes, after shave lotions, preparations such as bath oils and bath salts, hair preparations such as lacquers, brilliantines, pomades and shampoos, cosmetic preparations such as creams, deodorants, hand lotions and sun screens, powders such as talcs, dusting powders, face powders, masking agents, candles, incense products, household products such as bleaches, cleaners and in technical products such as shoe polish and automobile wax. Due to their advantageous and well-balanced properties, they are particularly suitable for use in compositions that are used under rather harsh conditions, i.e. in functional perfumery. This applies particularly to detergents, soaps, shampoos, household cleaners, fabric care products, and the like.

The macrocyclic oxalactones according to the invention can be prepared by several methods known to the art. Overviews of suitable preparation methods may be found in, inter alia, J. March, Advanced Organic Chemistry: Reactions, Mechanisms and Structure, 4^{th} edition, J. Wiley & Sons, New York, 1992, Appendix B, Ethers, pp. 1285-1286, and Lactones pp. 1292-1293 and R.C. Larock, Comprehensive Organic Transformations, A Guide to Functional Group Preparations, VCH Publishers, Inc., New York, 1998, Ethers, pp. 439-473 and Lactones, pp. 941-962, and more specifically for oxalactones e.g. in "Fragrance Chemistry" edited by E.T. Theimer, Academic Press 1982, p. 500-502 and references cited therein, in P.Z. Bedoukian, Perfumery and Flavoring Synthetics, 2^{nd} edition, Elsevier, 1967, pp. 259-265 and references cited therein and in P. Kraft et al. in Synthesis (11) pp. 1662-1669 (1998).

Preferably, the present macrocyclic oxalactones are prepared by starting from their corresponding diols and hydroxycarboxylic acids, or corresponding halogenated compounds. Preferred starting materials are:

X- (CH₂)ₙ-CR₁R₂ - (CH₂)ₘ-Y (II),

and

X- (CH₂)ₚ-CR₃R₄ - (CH₂)_{q}-X (III),

wherein each X is independently chosen from the group of leaving groups and protective groups, such as tosyl esters, silyl ethers, pyranyl ethers and the like, -OH, -Cl, -Br, -I, ether groups, ester groups and acetal groups, and wherein Y is a carboxyl group, or a salt, ester, anhydride, amide or acid halogenide (preferably an acid chloride) thereof, or a cyanide group. If an X group is an ether, ester or acetal group, said group will usually not contain more than ten carbon atoms. Likewise, if the Y group is an ester, amide or anhydride group, said group will generally not contain more than twelve carbon atoms. The other variables have the meanings specified above.

These preferred starting materials may be reacted to the objective macrocyclic oxalactone in any suitable manner known in the art of organic chemistry. The reaction may evolve through an intermediate of the formula:

X- (CH₂)_{q}-CR₃R₄ - (CH₂)ₚ-O- (CH₂)ₙ-CR₁R₂ - (CH₂)ₘ-Y (IV),

wherein the variables have the meanings specified above. Further intermediates that may be encountered are any reaction or polymerization products of one or more of the compounds of the formulas (II), (III) and (IV). A compound of the formula (II), together with one or more compounds of the formulas (III) and (IV), may react to form polyesters which are also encompassed by the invention.

Of the many general methods known for ether formation and for lactone formation, the preferred method will be specific for the representative of the novel compounds of the invention and will depend on considerations of economics, availability of starting materials, by-product formation, technical feasibility, safety, organoleptic grade produced, and the like, which parameters, circumstances and conditions may be subject to change over time, location, facility and so forth.

The following examples illustrate the invention without limitation thereto.

### Example 1

To a solution of 104 g 86% 12-chlorododecanol in 400 ml toluene was dosed with stirring 30.5 g sodium hydride at room temperature. The mixture was stirred for another hour at room temperature Then a solution of 35.9 g chloroacetic acid and 100 ml toluene was added gradually and the mixture was heated at reflux for 4 hours. After cooling down to 100 °C, 1000 ml of 5% sodium hydroxide solution was added. The organic layer was separated and combined with toluene and ethyl acetate extracts of the aqueous layer. Evaporation of the solvents gave 86 g of crude 15-chloro-3-oxapentadecanoic acid, 30 g of which was refluxed with 100 ml of 25% potassium hydroxide aqueous solution for 65 hrs. Acidification with 18% hydrochloric acid and extraction with ethyl acetate yielded after evaporation 31.5 g of crude 15-hydroxy-3-oxapentadecanoic acid. 175 g of this crude acid was dissolved in 243 g cumene and polymerized by heating with 0.6 g p-toluenesulphonic acid with removal of water formed. After removal of the cumene under vacuum by a rotatory evaporator, the residue was depolymerized by heating with 12.3 g glycerine and 6 g potassium carbonate at 0.5 mbar and 180 °C with removal by distillation of 54.5 g 1,4-dioxahexadecan-2-one. 1,4-Dioxahexadecan-2-one of 99.78% purity by GC was obtained by high vacuum fractionation through a Spaltrohr column, b.p. 100-105°C at 0.5 mbar, n_{D}²⁰ 1.4708.

### EXAMPLE 2

A mixture of 435.7 g 1,10-decanediol, 236.3 g chloroacetic acid and 800 ml toluene was heated at reflux for six hours with removal of the water formed by a Dean Stark trap till no more water was formed. The toluene was evaporated and the residue was taken up in 2.5 l toluene. To the mixture was carefully added at reflux and in small portions 100 g of 60% sodium hydride followed by a reflux period overnight. The mixture was quenched by the dropwise addition of 50 ml water and then concentrated by removal of 2 l toluene under reduced pressure. To the remainder was added successively 500 ml ethanol, 2 l 10% sodium hydroxide solution and 500 ml water and the resulting mixture was heated to reflux for an overnight period, cooled to room temperature, washed with 3 x 500 ml diethyl ether after the addition of 500 ml ethanol and 500 ml water and acidified with concentrated hydrochloric acid solution to pH=1. After separation of the phases the aqueous layer was extracted twice with 500 ml diethyl ether and the combined organic layers were washed twice with 250 ml water and concentrated by means of a rotatory evaporator yielding 383.5 g of crude product which was further purified by uptake in 10% sodium hydroxide solution, extraction with methyl-t-butyl ether and acidification yielding 254.6 g 13-hydroxy-3-oxatridecanoic acid of 95% purity by GC. This hydroxyacid was converted into the cyclic lactone analogously to the method set forth in Example 1 yielding 91.8 g crude 1,4-dioxatetradecan-2-one of 92% purity by GC. 1,4-Dioxatetradecan-2-one of 99.8 % purity by GC was obtained by high vacuum fractionation through a Spaltrohr column, bp 111°C at 3 mbar, n_{D}²⁰ = 1.4719.

### EXAMPLE 3

Analogously to the method set forth in Example 1, 286 g of 14-hydroxy-4-oxatetradecanoic acid was converted into the cyclic lactone yielding 130 g of crude 1,4-dioxapentadecan-2-one of 93% purity by GC. 1,4-Dioxapenta-decan-2-one of 99.7 % purity by GC was obtained by high vacuum fractionation through a Spaltrohr column, bp 94°C at 0.01 mbar, n_{D}²⁰ = 1.4716.

### EXAMPLE 4

To a mixture of 405 g 1,12-dodecanediol and 2 1 toluene was slowly added at reflux temperature 80 g of 60% sodium hydride. After heating at reflux for another hour 172 g of γ-butyrolactone was added slowly and the toluene was distilled off and the remainder was heated to 250 oC for 2 hours. After cooling to room temperature 500 ml ethanol was added followed by 100 g sodium hydroxide and 2.5 l water. The resulting mixture was heated till all material was dissolved, then cooled to roomtemperature, washed three times with 800 ml ethyl acetate and acidified with concentrated hydrochloric acid. After separation of the phases the aqueous layer was extracted twice with 400 ml ethyl acetate and the combined organic layers were washed twice with 500 ml water and concentrated by means of a rotatory evaporator yielding 368 g of crude product which was further purified by uptake in 10% sodium hydroxide solution, extraction with methyl-t-butyl ether and acidification yielding 288 g 17-hydroxy-5-oxaheptadecanoic acid of 95% purity by GC. This hydroxyacid was converted to the cyclic lactone analogously to the method set forth in Example 1 yielding 64.3 g crude 1,6-dioxaoctadecan-2-one of 93.4% purity by GC. 1,6-Dioxaoctadecan-2-one of 99.8% purity by GC was obtained by high vacuum fractionation through a Spaltrohr column, bp 113.5 °C at 0.02 mbar, n_{D}²⁰ = 1.4677.

### EXAMPLE 5

Analogously to the procedure set forth in Example 4 was prepared 265 g 15-hydroxy-5-oxapentadecanoic acid of 98% purity by GC from 300 g 1,10-decanediol and 148 g γ-butyrolactone. 162 g of this hydroxyacid was converted to the cyclic lactone analogously to the method set forth in Example 1 yielding 56.6 g crude 1,6-dioxahexadecan-2-one of 93.4% purity by GC. 1,6-Dioxahexadecan-2-one of 99.8% purity by GC was obtained by high vacuum fractionation through a Spaltrohr column, bp 85 °C at 0.01 mbar, n_{D}²⁰ = 1.4462.

### EXAMPLE 6

1,5-dioxaheptadecan-2-one of 93% purity by GC was prepared analogously to the method set forth in C.F.H. Allen and J.A. van Allen, J. Org. Chem. 14, 754-60 (1949) from 1.12-dodecanediol in 25.6% overall yield. 1,5-Dioxaheptadecan-2-one of 99.8% purity by GC was obtained by high vacuum fractionation through a Spaltrohr column, bp 119 °C at 0.02 mbar, n_{D}²⁰ = 1.4670.

### Example 7

Mixture A was prepared by admixing the following fragrance chemicals:

| Mixture A | |
|---|---|
| | Parts by weight |
| Melusat (Henkel) | 10 |
| Verdoxan (Henkel) | 25 |
| Lilial (Givaudan) | 50 |
| Methyl Ionone Alpha Extra (IFF) | 75 |
| Vertofix Coeur (IFF) | 75 |
| Alpha-Hexylcinnamic Aldehyde | 100 |
| Jasmacyclene (Quest) | 125 |
| Cyclohexyl Acetate | 150 |
| Vertenex (IFF) | 155 |
| | 765 |

The following test perfumes were prepared by admixing the following ingredients:

| Perfume B | |
|---|---|
| | Parts by weight |
| Mixture A | 75 |
| Dipropylene glycol | 15 |
| Compound of Example 1 | 10 |
| | 100 |

| Perfume C | |
|---|---|
| | Parts by weight |
| Mixture A | 75 |
| Dipropylene glycol | 15 |
| Galaxolide 50 (IFF)* | 10 |
| | 100 |

| | |
|---|---|
| * (1,3,4,6,7,8-hexahydro-4,6,6,7,8,8-hexamethylcyclopenta-γ-2benzopyran and isomers) | |

| Perfume D | |
|---|---|
| | Parts by weight |
| Mixture A | 75 |
| Dipropylene glycol | 15 |
| Habanolide (Firmenich)* | 10 |
| | 100 |

| | |
|---|---|
| (1-oxahexadec-4 and 5-en-2-one) | |

Perfumes B, C (containing a polycyclic musk) and D (containing a macrocyclic musk) were compared with respect to odor by a perfumers panel. Perfume B and C were found very close to each other, the musk notes of perfume B even being reminiscent of Tonalid® (PFW) (6-acetyl-1,1,2,4,4,7-hexamethyltetraline, a polycyclic musk). Perfume D was found quite different with a fruity, apple note.

### Example 8

Non-perfumed Compact detergent powder was perfumed on a 0.4% level with respectively the compound of Example 1 and 1,6-Dioxacycloheptadecan-7-one. Standard 40 °C main wash programs were performed with household cotton towels and cotton/synthetic cloths in a standard household washing machine, respectively applying these perfumed detergent powders respectively. After washing the laundry was evaluated on odor in the wet and dry stages by a perfumers panel. The odor of the laundry washed with the detergent powder containing the lactone of Example 1 was rated clear recognizable musk, with buttery, polycyclic musk connotations, reminiscent of Tonalid® (PFW) (6-acetyl-1,1,2,4,4,7-hexamethyltetraline, a polycyclic musk). The odor of the laundry washed with the detergent powder containing the 1,6-Dioxacycloheptadecan-7-one was rated clear recognizable with animalic, macrocyclic musk notes.

### Example 9

Non-perfumed Compact detergent powder was perfumed on a 0.4% level with respectively Perfumes B, C and D of Example 2. Standard 40 °C main wash programs were performed with household cotton towels and cotton/synthetic cloths in a standard household washing machine, respectively applying these perfumed detergent powders respectively. After washing the laundry was evaluated on odor in the wet and dry stages by a perfumers panel. The odor of the laundry washed with the detergent powder containing Perfume B was rated clear recognizable, clean, fresh, ozone and sweet musk, of the Tonalid® type (PFW) (6-acetyl-1,1,2,4,4,7-hexamethyltetraline, a polycyclic musk), with a cleaner impression than the laundry washed with Perfume C and D, the latter yielding a more dusty, fungal note.

## Claims

1. Macrocyclic oxalactone of the general formula (I) 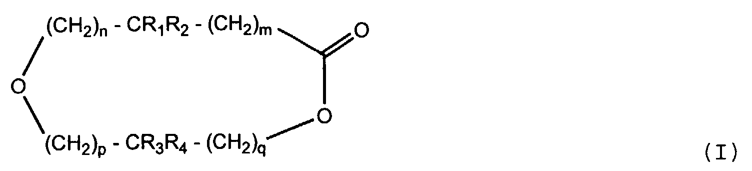 wherein
n and m represent an integer with the value 0, 1, 2, 3 or 4 the sum of n and m being 4 or less;
p and q represent an integer with the value 0 through 15 the sum of p and q being 15 or less;
the sum of n, m, p and q is 9, 10, 11, 12, 13, 14 or 15;
R₁, R₂, R₃ and R₄ independently represent hydrogen or a lower alkyl group
with the proviso that
when R₁, R₂, R₃ and R₄ all represent hydrogen:
p + q ≤ 11 if n + m = 0,
p + q = 11 if n + m = 1,
n + m + p + q ≠ 12 if n + m = 2,
n + m + p + q is not 11 or 12 if n + m = 3,
when R₁ represents a methyl group and R₂, R₃ and R₄ all represent hydrogen,
n + m + p + q ≠ 9 if n + m = 0.

2. Macrocyclic oxalactone according to claim 1, wherein R₁, R₂, R₃ and R₄ together do not comprise more than eight carbon atoms.

3. Macrocyclic oxalactone according to claim 2, wherein one of R₁ and R₂ is a hydrogen atom and one of R₃ and R₄ is a hydrogen atom.

4. Macrocyclic oxalactone according to claim 2 or 3, wherein the lower alkyl group is a methyl group.

5. Macrocyclic oxalactone according to claim 1, wherein R₁, R₂, R₃ and R₄ are hydrogen atoms.

6. Macrocyclic oxalactone according to any of the preceding claims, wherein the sum of n and m is zero and the sum of p and q is 9 or 10.

7. Macrocyclic oxalactone according to any of the preceding claims, wherein the sum of p and q is 11 and wherein the sum of n, m, p and q is 11, 12, 13, 14 or 15.

8. Macrocyclic oxalactone according to claim 7, wherein the sum of p and q is 11 and wherein the sum of n, m, p, and q is 11.

9. Macrocyclic oxalactone according to claim 8, wherein R₁, R₂, R₃ and R₄ all represent hydrogen.

10. Use of a macrocyclic oxalactone according to any of the preceding claims in functional perfumery.

11. Fragrance composition comprising a macrocyclic oxalactone according to claims 1-9.

12. Use of a fragrance composition according to claim 11 in a composition chosen from the group of detergents, soaps, shampoos, household cleaners, fabric care products, and the like.

13. Method for preparing a macrocyclic oxalactone according to claims 1-9, using as starting materials a compound of the formula X- (CH₂)ₙ-CR₁R₂ - (CH₂)ₘ-Y (II) and a compound of the formula X- (CH₂)_{P}-CR₃R₄ - (CH₂)_{q}-X, (III) wherein each X is independently chosen from group of leaving groups and protective groups, such as tosyl esters, silyl ethers, pyranyl ethers and the like, -OH, -Cl, -Br, -I, ether groups, ester groups and acetal groups; Y is a carboxyl group, or a salt, ester, anhydride, amide or acid halogenide (preferably an acid chloride) thereof, or a cyanide group; and the remaining variables have the meaning as specified in claim 1.

14. An intermediate compound of the formula
X- (CH₂)_{q}-CR₃R₄ - (CH₂)ₚ-O- (CH₂)ₙ-CR₁R₂ - (CH₂)ₘ-Y (IV),
wherein the variables have the meanings specified in claim 13.

15. An intermediate compound, being a reaction or polymerization product of one or more of the compounds of the formulas (II), (III) and (IV) as specified in claims 13 and 14.
